# EUROPEAN PATENT SPECIFICATION

(11) **EP 4 067 367 B1**
(45) Date of publication and mention of the grant of the patent: **28.05.2025**
(21) Application number: 20894763.0
(22) Date of filing: 26.11.2020
(51) Int. Cl.: C07J 63/00, A61K 31/56, A61P 25/28, A61P 35/00

(54) **OLEANANE CINNAMAMIDE DERIVATIVE, PREPARATION METHOD THEREFOR, AND USE THEREOF**
OLEANAN-CINNAMAMIDDERIVAT, VERFAHREN ZU SEINER HERSTELLUNG UND SEINE VERWENDUNG
DÉRIVÉ D'OLÉANANE CINNAMAMIDE, SON PROCÉDÉ DE PRÉPARATION ET SON UTILISATION

(30) Priority: 27.11.2019 CN 201911182967
(43) Date of publication of application: 05.10.2022
(73) Proprietor: Lunan Pharmaceutical Group Corporation, Linyi, Shandong 276006 (CN)
(72) Inventor: ZHANG, Guimin, Linyi, Shandong 276006 (CN); LIANG, Hongbao, Linyi, Shandong 276006 (CN); CHEN, Li, Nanjing, Jiangsu 210009 (CN); ZHAO, Guifang, Linyi, Shandong 276006 (CN); ZHAO, Zihao, Nanjing, Jiangsu 210009 (CN); LU, Xiaoyan, Linyi, Shandong 276006 (CN); SUN, Chenghong, Linyi, Shandong 276006 (CN)
(74) Representative: Meissner Bolte Partnerschaft mbB
(86) International application number: PCT/CN2020/131887
(87) International publication number: WO 2021/104391

(56) References cited:
- WO-A2-2009/146216
- AU-A1- 2017 356 955
- CN-A- 102 083 442
- CN-A- 105 517 554
- CN-A- 106 632 576
- US-A1- 2010 048 892

## Description

### FIELD OF TECHNOLOGY

The present invention relates to the fields of medicinal chemistry and pharmacotherapeutics, comprising a 2-cyano-3,12-dioxoolean-1,9(11)-dien-17-phenylacrylamide compound and a preparation method thereof; and the present invention further relates to use of the novel compound in the preparation of an anticancer medicament.

### BACKGROUND

Oleanolic acid (OA), a pentacyclic triterpenoid, is a natural product widely distributed in the plant kingdom and is the active ingredient of many traditional Chinese medicines, having a wide range of biological activities. OA-based structural optimization has always been one of the hot topics in natural medicinal chemical research. 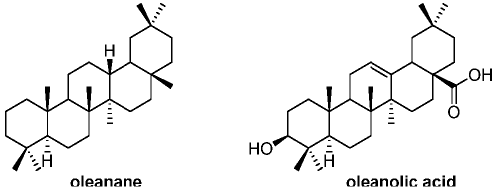 US 2010/048892 A1 discloses oleanolic acid derivatives useful in the treatment and prevention of diseases.

CDDO and derivatives thereof (CDDOs) are semi-synthetic OA derivatives with the strongest anti-tumor and anti-inflammatory activities found to date. In contrast to single-target medicaments, CDDOs possess properties including multi-function, multi-target and acting on the entire cell signaling pathways, and can exert anti-tumor effects in a plurality of processes, such that these derivatives have advantages of high activity and less incidence of drug resistance. Among the CDDOs, the methyl ester derivative of CDDO (2-cyano-3,12-dioxoolean-1,9(11)-dien-28-oic acid methyl ester, CDDO-Me), also known by the trade name Bardoxolone Methyl, was studied in 2006 in phase I clinical trials for the treatment of advanced solid tumors and lymphoid malignancies (NCT00529438; NCT00508807). In addition, CDDO-Me was evaluated for treatment of chronic kidney disease (CUK) caused by type II diabetes mellitus in a study approved by the FDA in 2008 (NCT00664027). But unfortunately, the evaluation was terminated in November 2013 during a phase III clinical study due to high lethality caused by heart failure. It was also reported that the medicament was used, as approved by the FDA in 2014, in a clinical study on pulmonary arterial hypertension (orphan medicament), which is currently at the stage of a phase III clinical study (phase II NCT02036970; phase III NCT02657356).

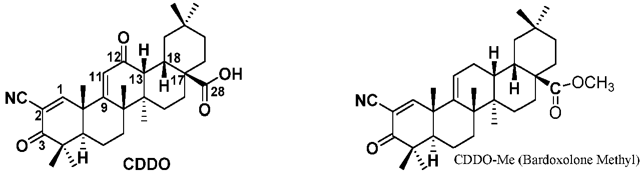

Although CDDO-Me has good anti-tumor activity, the myocardial toxicity thereof remains a concern. It would be of great practical significance to retain or enhance the activity of the compounds while reducing the toxicity thereof through necessary modification and reform.

### SUMMARY

The present invention discloses for the first time a 2-cyano-3,12-dioxoolean-1,9(11)-dien-17-phenylacrylamide compound, a method for preparing the same, and a medical use thereof. The preparation method of the derivatives of the present invention has the following advantages: raw materials are cheap and easily accessible; reagents are environmentally friendly and low toxic; conditions are mild and easy to control; post-treatment are convenient and simple; and meanwhile the preparation method is practical and universal. According to the results of pharmacological experiments, the compounds of the present invention have excellent anti-tumor activity which is higher than or comparable to the anti-tumor activity of CDDO-Me; and the myocardial toxicity of some of the compounds is significantly reduced. Therefore, the compounds can be candidate compounds for an anti-tumor medicament.

One objective of the present invention is to provide a 2-cyano-3, 12-dioxoolean-1,9(11)-dien-17-phenylacrylamide compound of formula I: 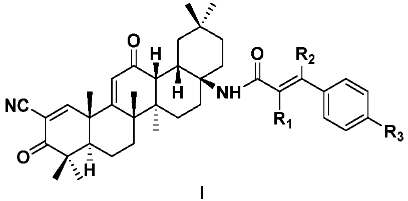 wherein R₁, R₂ and R₃ are respectively and independently selected from the following substituents: H, C₁₋₃ alkyl, alkoxy, halogen, cyano, C₁₋₃ alkyl substituted by halogen or cyano.

The halogen is selected from F, Cl, Br, and I.

The alkoxy is an alkoxy containing 1-3 carbon atoms.

In some embodiments, the R₁ and R₂ of the above formula I are respectively and independently selected from H or cyano; and R₃ is selected from H, C₁₋₃ alkyl, alkoxy or halogen.

In some embodiments, the R₁ and R₂ of the above formula I are respectively and independently selected from H or cyano; and R₃ is selected from F, Cl, H, and methoxy.

In some embodiments, the R₁ and R₂ of the above formula I are respectively and independently selected from H or cyano; and R₃ is selected from Cl and H.

In some embodiments, R₁ of the above formula I is cyano; R₂ is H; and R₃ is Cl or H.

In another aspect, the present invention provides a compound of formula I hereinafter or a pharmaceutically acceptable salt thereof: 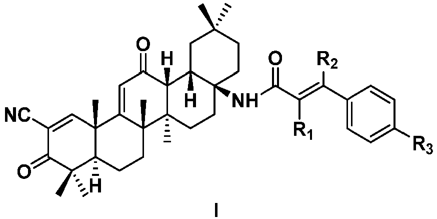 wherein R₁, R₂ and R₃ are respectively and independently selected from the following substituents: H, C₁₋₆ alkyl, C₁₋₆ alkoxy, halogen, cyano, C₁₋₆ alkyl substituted by halogen or cyano; preferably H, C₁₋₃ alkyl, C₁₋₃ alkoxy, halogen, cyano, C₁₋₃ alkyl substituted by halogen or cyano.

In some embodiments, the R₁ and R₂ of the above formula I are respectively and independently selected from H or cyano; R₃ is selected from H, C₁₋₃ alkyl, alkoxy, halogen, C₁₋₃ alkyl substituted by halogen; the alkoxy is an alkoxy containing 1-3 carbon atoms; and the halogen is selected from F, Cl, Br, and I.

In some embodiments, the R₁ of the above formula I is H or cyano; R₂ is H or cyano; and R₃ is selected from H, methyl, methoxy, trifluoromethyl, Cl or F.

In some embodiments, the R₁ of the above formula I is H or cyano; R₂ is H or cyano; and R₃ is Cl, H or trifluoromethyl.

In some embodiments, the compound of formula I is selected from the following compounds:

| **Cpd** | **R₁** | **R₂** | **R₃** |
|---|---|---|---|
| I-1 | H | H | H |
| I-2 | H | H | CH₃ |
| I-3 | H | H | OCH₃ |
| I-4 | H | H | CF₃ |
| I-5 | H | H | F |
| I-6 | H | H | Cl |
| I-7 | CN | H | H |
| I-8 | CN | H | CH₃ |
| I-9 | CN | H | OCH₃ |
| I-10 | CN | H | CF₃ |
| I-11 | CN | H | F |
| I-12 | CN | H | Cl |
| I-13 | H | CN | H |
| I-14 | H | CN | CH₃ |
| I-15 | H | CN | OCH₃ |
| I-16 | H | CN | CF₃ |
| I-17 | H | CN | F |
| I-18 | H | CN | Cl |

Further provided in the present invention is a preparation method of the compound of formula I, comprising the following steps: 1. subjecting CDDO to Curtius rearrangement to obtain a derivative (CDDO-NH₂) aminated at the C-17 position, the structure being shown by formula II:

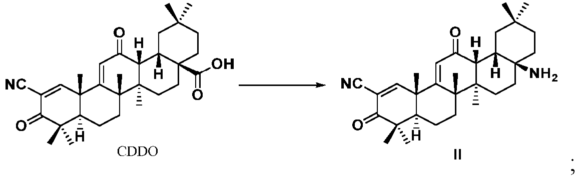

2. reacting formula II with formula III (a phenylacrylic acid compound) via a condensation reaction to obtain a compound of formula I: 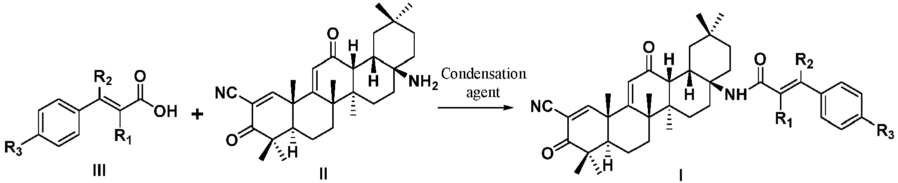
wherein the condensation agent is one or more selected from dicyclohexylcarbodiimide, *N*,*N*-diisopropylcarbodiimide, 1-ethyl-(3-dimethylaminopropyl) carbodiimide, benzotriazol-1-yloxytris(dimethylamino)phosphonium hexafluorophosphate or benzotriazol-1-yloxytripyrrolidinophosphonium hexafluorophosphate;
the solvent may be one or more selected from, but not limited to, *N*,*N*-dimethylformamide, acetone, acetonitrile, toluene, benzene, dimethylbenzene, 1,4-dioxane, ethyl acetate, dichloromethane, chloroform, tetrahydrofuran or ether; and preferably the reaction temperature is between 0°C and 60°C.

A further objective of the present invention is to provide a pharmaceutical composition manufactured by and containing an effective dose of the compound of the present invention or a pharmaceutically acceptable salt thereof, and one or more pharmaceutically acceptable pharmaceutical adjuvants. The compound of the present invention can be manufactured, either individually or with one or more pharmaceutical carriers, into various dosage forms such as tablets, capsules, granules, liquid pharmaceutical preparations, etc., for clinical oral administration, injection or local applications. In these various formulations, the quantity of the compound of the present invention may range from 0.1% to 99.9%. The compound of the present invention may be administered at a dose volume of from 0.001 to 10000 mg/kg per 0.3 days, which may be moderately adjusted according to clinical needs.

The "pharmaceutically acceptable salt" of the present invention refers to a pharmaceutically acceptable acid or base addition salt, or a solvate thereof.

Another objective of the present invention is to provide use of the compound or a pharmaceutically acceptable salt thereof and the pharmaceutical composition thereof in the preparation of an anticancer medicament. The tumor is selected from lung cancer, liver cancer, colon cancer, pancreatic cancer, breast cancer, prostatic cancer, brain cancer, ovarian carcinoma, cervical cancer, testicular cancer, kidney cancer, head and neck cancer, lymphoma, melanoma or leukemia. Further, the tumor is selected from lung cancer, liver cancer or breast cancer. Preferably, the tumor is lung cancer. According to the results of a series of tumor cell tests, the compounds of the present invention have a broad-spectrum anti-tumor activity with IC₅₀ values thereof all in the nanomolar to micromolar range, comparable to the activity of the positive control medicament CDDO-Me. Among the compounds, compounds I-7 and I-12 have a toxicity to rat embryonic cardiomyocytes H9C2 (IC₅₀ values of 3.176±1.74 µM and 3.143±1.53 µM, respectively) significantly lower than that of CDDO-Me (IC₅₀ of 0.308±0.01 µM).

Another objective of the present invention is to provide use of the compound or a pharmaceutically acceptable salt thereof and a pharmaceutical composition thereof in the preparation of an anticancer medicament. The tumor is selected from lung cancer, liver cancer, ascitic tumor, brain metastasis, colon cancer, pancreatic cancer, breast cancer, prostatic cancer, brain cancer, ovarian carcinoma, cervical cancer, testicular cancer, kidney cancer, head and neck cancer, lymphoma, melanoma or leukemia; preferably selected from lung cancer, liver cancer, breast cancer, ascitic tumor, pancreatic cancer, brain metastasis; and further preferably selected from non-small cell lung cancer, liver cancer, breast cancer, and ascitic tumor. According to the results of a series of tumor cell tests, the compounds of the present invention have a broad-spectrum anti-tumor activity with IC₅₀ values all in the nanomolar to micromolar range, comparable to the activity of the positive control medicament CDDO-Me. Among the compounds, compounds I-7 and I-12 have a toxicity to rat embryonic cardiomyocytes H9C2 (IC₅₀ values of 3.176±1.74 µM and 3.143±1.53 µM, respectively) significantly lower than the toxicity of CDDO-Me (IC₅₀ of 0.308±0.01 µM); moreover, compounds I-1, I-2, I-3, I-4, I-5, I-6, I-7, I-8, I-9, I-12, I-13, I-14, I-16 and I-18 all exhibited lower toxicity to human renal tubular epithelial cells HK-2. Another further objective of the present invention is to provide the compound or a pharmaceutically acceptable salt thereof, and a pharmaceutical composition thereof for use against tumor. The tumor is selected from lung cancer, liver cancer, ascitic tumor, brain metastasis, colon cancer, pancreatic cancer, breast cancer, prostatic cancer, brain cancer, ovarian carcinoma, cervical cancer, testicular cancer, kidney cancer, head and neck cancer, lymphoma, melanoma or leukemia; preferably selected from lung cancer, liver cancer, breast cancer, ascitic tumor, pancreatic cancer, and brain metastasis; and further preferably selected from non-small cell lung cancer, liver cancer, breast cancer, and ascitic tumor. According to the results of a series of tumor cell tests, the compounds of the present invention have a broad-spectrum anti-tumor activity with IC₅₀ values all in the nanomolar to micromolar range, comparable to the activity of the positive control medicament CDDO-Me. Among the compounds, compounds I-7 and I-12 have a toxicity to rat embryonic cardiomyocytes H9C2 (IC₅₀ values of 3.176±1.74 µM and 3.143±1.53 µM, respectively) significantly lower than the toxicity of CDDO-Me (IC₅₀ of 0.308±0.01 µM); moreover, compounds I-1, I-2, I-3, I-4, I-5, I-6, I-7, I-8, I-9, I-12, I-13, I-14, I-16 and I-18 all exhibited lower toxicity to human renal tubular epithelial cells HK-2.

An even further objective of the present invention is to provide compounds for use in treating tumor, comprising administering to a subject or patient a therapeutically effective dose of the compound or a pharmaceutically acceptable salt thereof and the pharmaceutical composition thereof. The tumor is selected from lung cancer, liver cancer, ascitic tumor, brain metastasis, colon cancer, pancreatic cancer, breast cancer, prostatic cancer, brain cancer, ovarian carcinoma, cervical cancer, testicular cancer, kidney cancer, head and neck cancer, lymphoma, melanoma or leukemia; preferably selected from lung cancer, liver cancer, breast cancer, ascitic tumor, pancreatic cancer, and brain metastasis; and further preferably selected from non-small cell lung cancer, liver cancer, breast cancer, and ascitic tumor. According to the results of a series of tumor cell tests, the compounds of the present invention have a broad-spectrum anti-tumor activity with IC₅₀ values all in the nanomolar to micromolar range, comparable to the activity of the positive control medicament CDDO-Me. Among the compounds, compounds I-7 and I-12 have a toxicity to rat embryonic cardiomyocytes H9C2 (IC₅₀ values of 3.176±1.74 µM and 3.143±1.53 µM, respectively) significantly lower than the toxicity of CDDO-Me (IC₅₀ of 0.308±0.01 µM); moreover, compounds I-1, I-2, I-3, I-4, I-5, I-6, I-7, I-8, I-9, I-12, I-13, I-14, I-16 and I-18 all exhibited lower toxicity to human renal tubular epithelial cells HK-2.

Even though preferred embodiments of the present invention have been displayed and described herein, it is obvious for those skilled in the art that such kind of embodiment is merely provided by examples. Now, a person skilled in the art will envisage lots of changes, variations and replacements not departing from the present invention. It should be understood that various replacements in embodiments of the present invention described herein may be used for implementing the present invention. The claims annexed are aimed at defining the scope of the present invention such that methods, structures and equivalent forms thereof within the scope of these claims are covered.

### Some chemical terms

Unless otherwise specified, all the scientific and technological terms herein have the meanings the same as those generally understood by a person skilled in the art of the subject of the claims.

It should be understood that the above sketch and the following detailed description are exemplary and only used for explanation, but not construed as limiting the subject of the present application. Unless otherwise specified, a plural form is also included in case of using the singular in this present application. It should be further noted that unless otherwise specified, the "or" and "either" denote "and/or". Furthermore, the term "comprise" and other forms thereof used herein, for example, "include", "contain" and "have" are non-restrictive descriptions.

Definitions on standard chemical terms may be found in references (including Carey and Sundberg "ADVANCED ORGANIC CHEMISTRY 4TH ED." Vols. A (2000) and B (2001), Plenum Press, New York). Unless otherwise specified, conventional methods within the technical scope of the art, for example, mass spectrometry, NMR, IR and UV/Vis spectroscopy and pharmacological methods, are employed. Unless specifically defined, terms used herein in the related description of analytical chemistry, organic synthetic chemistry, medicine and medicinal chemistry are known in the art. Standard technologies may be used in chemical synthesis, chemical analysis, medicament preparation, formulation and delivery For example, reaction and purification may be implemented by means of a manufacturer's instruction for a kit, or according to a way commonly known in the art or by the description of the present application. Generally, the above technology and method may be implemented according to the description of multiple summary and more detailed literatures cited and discussed in this description, by a conventional method known very well in the art. In this description, a person skilled in the art may choose a group and a substituent thereof to provide a stable structural portion and a compound.

When a substituent is described by a conventional chemical formula written from left to right, the substituent also includes a chemically equivalent substituent obtained by writing a chemical formula from right to left. For example, CH₂O is equivalent to OCH₂.

The "compound" described herein refers to including all the stereoisomers, geometrical isomers, tautomers and isotopes. The compound of the present application may be asymmetrical, for example, having one or more stereoisomers. Unless otherwise specified, all the stereoisomers include, for example, enantiomers and diastereoisomers. The compound having asymmetrically substituted carbon atoms in the present application may be separated in an optically active pure form or in a racemic form. The optically active pure form may be split from a racemic mixture, or synthesized by a chiral raw material or a chiral reagent. The compound of the present application further includes a form of a tautomer. The form of a tautomer is derived from an exchange of a single bond and an adjacent double bond accompanied with migration of a proton. The compound of the present application further includes atoms of all the isotopes, whether it is in an intermediate or a final compound. Atoms of isotopes include having the same atomic number but a different mass number. For example, isotopes of H include deuterium and tritium. That is, the compound of the present application includes compounds whose partial or complete hydrogen (H) atoms are replaced by tritium (T) and/or deuterium (D); and also includes compounds whose partial or complete ¹²C are replaced by ¹³C and/or ¹⁴C; as well as compounds whose atoms of other elements (e.g., N, O, P and S) are replaced by isotopes thereof, for example, ¹⁴N and ¹⁵N; ¹⁸O and ¹⁷O; ³¹P and ³²P; ³⁵S and ³⁶S, and the like. The compound described herein may have one or more stererogenic centers; and each stererogenic center may exist in a R or an S configuration or a combination thereof. Similarly, the compound described herein may have one or more double bonds; and each double bond may exist in an E (*trans*-) or Z (*cis*-) configuration or a combination thereof. A specific stereoisomer, constitutional isomer, diastereoisomer, enantiomer or epimer should be construed as including all the possible isomers, such as, stereoisomers, constitutional isomers, diastereoisomers, enantiomers or epimers, and a mixture thereof. Therefore, the compound described herein includes all the configurationally different stereoisomers, constitutional isomers, diastereoisomers, enantiomers or epimers, and the corresponding mixtures thereof. The technology used for transforming a specific stereoisomer or keeping a specific stereoisomer the same and the technology for splitting a mixture of stereoisomers are well known in the art, and a person skilled in the art is able to choose a proper method for a specific condition.

The term "optional/arbitrary" or "optionally/arbitrarily" refers to that the subsequently described event or situation may or may not occur, and the description includes occurrence of the event or situation and no occurrence of the event or situation.

The C₁₋₃ used herein denotes that the part has 1-3 carbon atoms, i.e., the group contains 1 carbon atom, 2 carbon atoms, 3 carbon atoms. Therefore, for example, "C₁-C₄ alkyl" refers to an alkyl containing from 1-4 carbon atoms, i.e., the alkyl is selected from methyl, ethyl, propyl, isopropyl, n-butyl, isobutyl, sec-butyl and tert-butyl.

The term "alkyl" used herein, individually or combined, refers to optionally substituted linear aliphatic hydrocarbons or optionally substituted branched aliphatic hydrocarbons. Preferably, "alkyl" herein may have from 1 to about 20 carbon atoms, such as having from 1 to about 10 carbon atoms, having from 1 to about 8 carbon atoms, or from 1 to about 6 carbon atoms, or from 1 to about 4 carbon atoms, or from 1 to about 3 carbon atoms. Examples of the alkyl herein include, but are not limited to, methyl, ethyl, n-propyl, isopropyl, etc. Groups defined herein, such as "alkyl", where a numerical range appears, e.g. "C₁-C₆ alkyl" or "C₁₋₆ alkyl" refers to an alkyl which may consist of 1 carbon atom, 2 carbon atoms, 3 carbon atoms, 4 carbon atoms, 5 carbon atoms, or 6 carbon atoms, and the alkyl herein also includes a situation where no numerical range is specified.

The "alkyl" used herein combined includes an alkyl in combination with other groups, such as alkyl in alkoxy.

The term "halogen" used herein, individually or combined, is selected from F, Cl, Br and I.

The term "halogenated" or "halogen-substituted" used herein, individually or combined, denotes that one or more hydrogen atoms in an optionally substituted group (e.g., alkyl, alkenyl, and alkynyl) are replaced by an atom of fluorine, chlorine, bromine, iodine, or a combination thereof. In some embodiments, two or more hydrogen atoms are replaced by halogen atoms which are identical to each other (e.g., difluoromethyl, trifluoromethyl); in other embodiments, two or more hydrogen atoms are replaced by halogen atoms which are not absolutely identical to each other (e.g., 1-chloro-1-fluoro-1-iodoethyl). Non-limiting examples of haloalkyl are fluoromethyl and bromoethyl. A non-limiting example of haloalkenyl is bromovinyl. A non-limiting example of haloalkynyl is chloroethynyl.

The term "treat" and other similar synonyms used herein include relieving, alleviating or improving a symptom of a disease or a condition, inhibiting a disease or a condition, for example, blocking the progression of a disease or a condition, relieving a disease or a condition, making a disease or a condition improved, relieving a symptom caused by a disease or a condition, or suspending a symptom of a disease or a condition, preventing other symptoms, improving or preventing a potential metabolism cause for a symptom; further, the term includes a purpose of prevention. The term further includes obtaining a therapeutic effect and/or prevention effect. The therapeutic effect refers to healing or improving a potential disease which is treated. Furthermore, healing or improving one or more physiological symptoms related to a potential disease is also a therapeutic effect, for example, even though a patient may be still influenced by a potential disease, improvement in the condition of the patient is observed. In term of a prevention effect, a patient having the risk of suffering from a specific disease is administered the composition, or the composition is administered to a patient with one or more physiological symptoms of the disease even if no disease diagnosis is made yet.

The term "effective dose", "therapeutically effective dose" or "pharmaceutically effective dose" used herein refers to an administered dose of at least one active substance (e.g., the compound of the present application) which is enough to relieve to some extent one or more physiological symptoms of the disease or condition after a treatment. The result may be reduction and/or remission of a sign, a symptom or a cause for a disease, or any other change required in a biosystem. For example, the "effective dose" for a treatment refers to a dose of the composition containing the compound disclosed herein required to provide a significant disease/condition relieving effect clinically. A technology, for example, a dose escalation trial, may be used to determine an effective dose suitable for any individual case.

The term "acceptable" used herein, specific to the formulation, composition or component, denotes that there is no long-term deleterious effect on the general health condition of a subject receiving a treatment.

The term "pharmaceutically acceptable" used herein refers to a substance (e.g., a carrier or a diluent) which does not affect the biological activity or properties of the compound of the present application, and is relatively non-toxic; namely, the substance may be applied to an individual without causing an adverse biological response or interacting with any component contained in the composition in an adverse manner.

The term "pharmaceutical composition" used herein refers to a mixture of the compound of the present application and at least one pharmaceutically acceptable substance. The pharmaceutically acceptable substance includes but not limited to a carrier, a stabilizer, a diluent, a dispersant, a suspending agent, a thickener and/or an excipient.

The term "carrier" used herein refers to a relatively non-toxic substance which helps to introduce the compound of the present application into a cell or tissue.

The term "pharmaceutically acceptable salt" used herein refers to a salt which retains the biological efficacy of a free acid and free alkali of a designated compound, and is free of adverse effects in biology or other aspects. The compound of the present application further includes a pharmaceutically acceptable salt. The pharmaceutically acceptable salt refers to conversion of a base group in a parent compound into a salt form. The pharmaceutically acceptable salt includes but not limited to an inorganic or organic acid salt of a basic group like amido (amino). The pharmaceutically acceptable salt of the present application may be synthesized by a parent compound, namely, a basic group in a parent compound being reacted with 1-4 equivalent amount of an acid in a solvent system. A proper salt is enumerated in Remingtong's Pharmaceutical Sciences, 17th ed., Mack Publishing Company, Easton, Pa., 1985, p. 1418, and Journal of Pharmaceutical Science, 66, 2 (1977).

Unless otherwise specified, the salt in the present application refers to an acidic salt formed by an organic/inorganic acid, and a basic salt formed by an organic/inorganic base. Additionally, a zwitterion (inner salt) will be formed when the basic functional group of the compound in the general formula is pyridine or imidazole (but not limited thereto) and the acidic functional group is carboxylic acid (but not limited thereto); and the inner salt is also included in the salt of the present application.

### DETAILED DESCRIPTION OF THE EMBODIMENTS

A series of examples are enumerated herein in order to give a clearer description of the present invention. These examples are exemplary and should not be construed as limiting the present invention.

### Example 1: Synthesis of I-1

0.080 g (0.170 mmol) of CDDO-NH₂ was dissolved in 5 mL of dichloromethane, to which 0.046 g (0.260 mmol) of 3-phenylacrylic acid was added and stirred, then 0.177 g (0.340 mmol) of benzotriazol-1-yloxytripyrrolidinophosphonium hexafluorophosphate (PyBop) and 0.074 mL (0.425 mmol) of *N,N-*diisopropylethylamine were added to react at room temperature for 12 h. Following the completion of the reaction as determined by thin layer chromatography (TLC), 5 mL of water and 5 mL of dichloromethane were added for extraction and separation, then the aqueous layer was further extracted with 5 mL of dichloromethane and the organic layers were combined. The organic layer was washed with 5 mL of saturated sodium chloride and dried with anhydrous sodium sulfate. Following suction filtration, the organic layer was evaporated under reduced pressure, subjected to column chromatography and rotary evaporation such that 0.036 g of a yellow-white solid was obtained with a yield of 44.9%.

ESI-MS: 593.4 [M+H]⁺.

¹H-NMR (300 MHz, CDCl₃, TMS), *δ* ppm: 0.89 (3H, s), 1.04 (3H, s), 1.05 (3H, s), 1.17 (3H, s), 1.26 (3H, s), 1.42 (3H, s), 1.47 (3H, s), 6.01 (1H, s), 6.53 (1H, d, *J* = 15.60 Hz), 7.35 (3H, m), 7.48 (2H, m), 7.60 (1H, d, *J* = 15.54 Hz), 8.08 (1H, s).

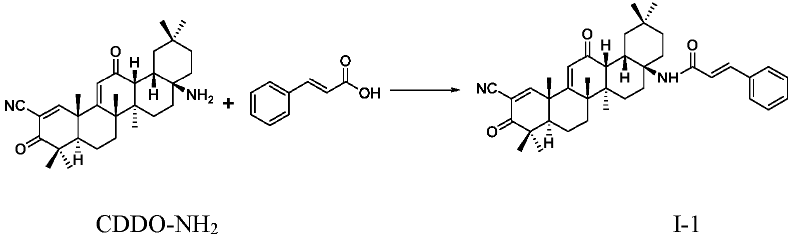

### Example 2: Synthesis of I-2

By reference to the preparation method of compound I-1 and substitution of 3-(4-methylphenyl)acrylic acid for 3-phenylacrylic acid as the reaction reagent while other conditions remained unchanged, 0.024 g of the target compound I-2, a pale yellow solid, was obtained with a yield of 28.8%.

ESI-MS: 607.4 [M+H]⁺.

¹H-NMR (300 MHz, CDCl₃, TMS), *δ* ppm: 0.90 (3H, s), 1.05 (6H, s), 1.17 (3H, s), 1.27 (3H, s), 1.41 (3H, s), 1.47 (3H, s), 2.36 (3H, s), 6.01 (1H, s), 6.43 (1H, d, *J* = 12.60 Hz), 7.19 (2H, s), 7.38 (2H, s), 7.57 (1H, d, *J* = 15.27 Hz), 8.06 (1H, s).

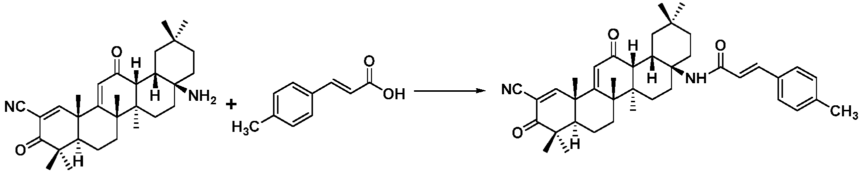

### Example 3: Synthesis of I-3

By reference to the preparation method of compound I-1 and substitution of 3-(4-methoxyphenyl)acrylic acid for 3-phenylacrylic acid as the reaction reagent while other conditions remained unchanged, 0.035 g of the target compound I-3, a yellow-white solid, was obtained with a yield of 41.2%.

ESI-MS: 623.4 [M+H]⁺, 645.4 [M+Na]⁺.

¹H-NMR (300 MHz, CDCl₃, TMS), *δ* ppm: 0.92 (3H, s), 1.06 (6H, s), 1.18 (3H, s), 1.27 (3H, s), 1.42 (3H, s), 1.48 (3H, s), 3.84 (3H, s), 6.01 (1H, s), 6.90 (1H, d, *J* = 8.49 Hz), 7.45 (1H, d, *J=* 8.43 Hz), 7.58 (4H, m), 8.05 (1H, s).

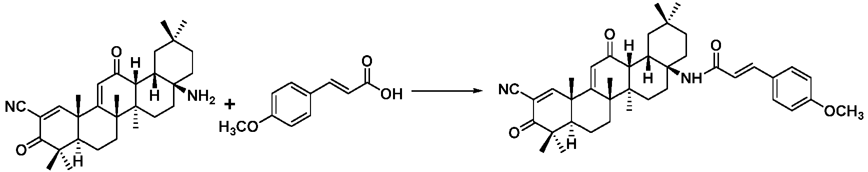

### Example 4: Synthesis of I-4

By reference to the preparation method of compound I-1 and substitution of 3-(4-trifluoromethylphenyl)acrylic acid for 3-phenylacrylic acid as the reaction reagent while other conditions remained unchanged, 0.018 g of the target compound I-4, a pale yellow solid, was obtained with a yield of 20.4%.

ESI-MS: 661.4 [M+H]⁺, 683.4 [M+Na]⁺.

¹H-NMR (300 MHz, CDCl₃, TMS), *δ* ppm: 0.91 (3H, s), 1.06 (3H, s), 1.07 (3H, s), 1.18 (3H, s), 1.27 (3H, s), 1.44 (3H, s), 1.49 (3H, s), 6.03 (1H, s), 6.54 (1H, d, *J* = 15.30 Hz), 7.61 (4H, m), 7.63 (1H, d, *J* = 12.96 Hz), 8.09 (1H, s).

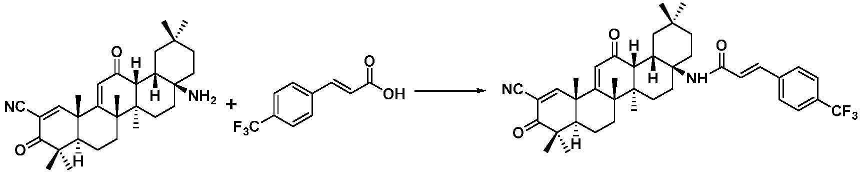

### Example 5: Synthesis of I-5

By reference to the preparation method of compound I-1 and substitution of 3-(4-fluorophenyl)acrylic acid for 3-phenylacrylic acid as the reaction reagent while other conditions remained unchanged, 0.019 g of the target compound I-5, a pale yellow solid, was obtained with a yield of 23.4%.

ESI-MS: 611.4 [M+H]⁺.

¹H-NMR (300 MHz, CDCl₃, TMS), *δ* ppm: 0.89 (3H, s), 1.05 (3H, s), 1.06 (3H, s), 1.17 (3H, s), 1.26 (3H, s), 1.45 (3H, s), 1.49 (3H, s), 6.01 (1H, s), 6.55 (1H, d, *J* = 8.58 Hz), 7.59 (2H, s), 7.64 (2H, s), 7.63 (1H, d, *J* = 8.61 Hz), 8.09 (1H, s).

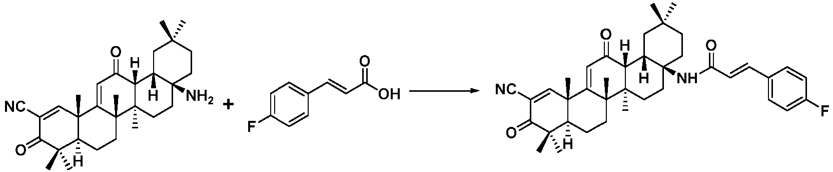

### Example 6: Synthesis of I-6

By reference to the preparation method of compound I-1 and substitution of 3-(4-chlorophenyl)acrylic acid for 3-phenylacrylic acid as the reaction reagent while other conditions remained unchanged, 0.025 g of the target compound I-6, a yellow-white solid, was obtained with a yield of 29.1%.

ESI-MS: 627.3 [M+H]⁺.

¹H-NMR (300 MHz, CDCl₃, TMS), *δ* ppm: 0.90 (3H, s), 1.05 (3H, s), 1.06 (3H, s), 1.18 (3H, s), 1.27 (3H, s), 1.44 (3H, s), 1.49 (3H, s), 6.03 (1H, s), 6.53 (1H, d, *J* = 8.66 Hz), 7.62 (2H, s), 7.68 (2H, s), 7.61 (1H, d, *J* = 8.64 Hz), 8.09 (1H, s).

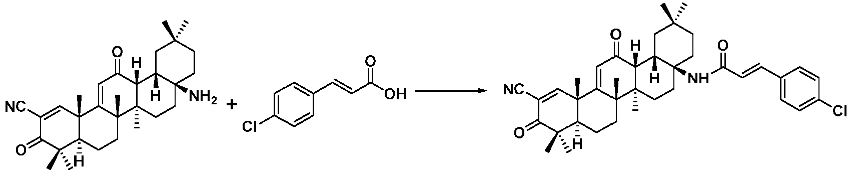

### Example 7: Synthesis of intermediate III-1 2-cyano-3-phenylacrylic acid

0.425 g (5 mmol) of cyanoacetic acid and 0.477 g (4.5 mmol) of benzaldehyde were dissolved with 20 mL of toluene, followed by addition of 0.058 g (0.75 mmol) of ammonium acetate. The mixture was heated under reflux to react for from 6-7 h, the completion of the reaction being determined by TLC. After cooling to room temperature, suction filtration was carried out, and the filter cake was vacuum dried to obtain 0.643 g of intermediate III-1, a white solid, with a yield of 82.4%.

ESI-MS: 174.0 [M+H]⁺.

The reaction formula is as follows:

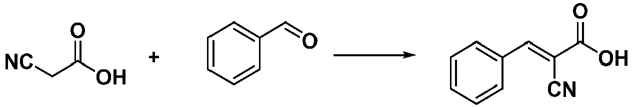

### Example 8: Synthesis of I-7

By reference to the preparation method of compound I-1 and substitution of 2-cyano-3-phenylacrylic acid for 3-phenylacrylic acid as the reaction reagent while other conditions remained unchanged, 0.026 g of the target compound I-7, a pale yellow solid, was obtained with a yield of 30.7%.

ESI-MS: 618.4 [M+H]⁺, 640.4 [M+Na]⁺, 616.4 [M-H]⁻.

¹H-NMR (300 MHz, CDCl₃, TMS), *δ* ppm: 0.93 (3H, s), 1.06 (3H, s), 1.13 (3H, s), 1.18 (3H, s), 1.27 (3H, s), 1.34 (3H, s), 1.49 (3H, s), 6.01 (1H, s), 7.35 (3H, m), 7.48 (2H, m), 8.06 (1H, s), 8.21 (1H, s).

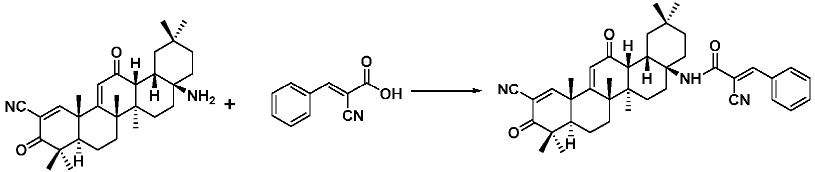

### Example 9: Synthesis of intermediate III-2 2-cyano-3-(4-methylphenyl)acrylic acid

By reference to the synthesis method of intermediate III-1 2-cyano-3-phenylacrylic acid and substitution of 4-methylbenzaldehyde for benzaldehyde while other conditions remained unchanged, 0.727 g of intermediate III-2 was obtained with a yield of 86.3%.

ESI-MS: 188.0 [M+H]⁺.

The reaction formula is as follows:

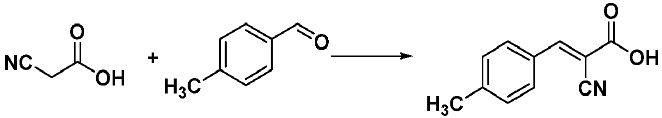

### Example 10: Synthesis of I-8

By reference to the preparation method of compound I-1 and substitution of 2-cyano-3-(4-methylphenyl)acrylic acid for 3-phenylacrylic acid as the reaction reagent while other conditions remained unchanged, 0.031 g of the target compound I-8, a pale yellow solid, was obtained with a yield of 35.9%.

ESI-MS: 632.4 [M+H]⁺, 654.4 [M+Na]⁺, 630.4 [M-H]⁻.

¹H-NMR (300 MHz, CDCl₃, TMS), *δ* ppm: 0.93 (3H, s), 1.06 (3H, s), 1.13 (3H, s), 1.18 (3H, s), 1.27 (3H, s), 1.34 (3H, s), 1.49 (3H, s), 2.43 (3H, s), 6.01 (1H, s), 6.99 (2H, d, *J* = 8.48 Hz), 7.92 (2H, d, *J* = 8.61 Hz), 8.06 (1H, s), 8.21 (1H, s).

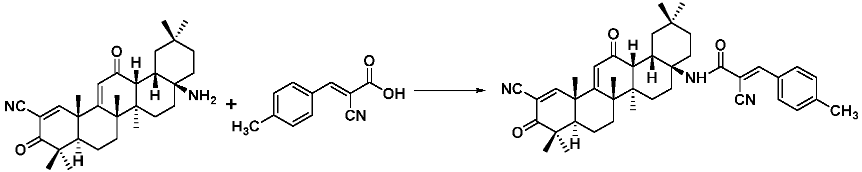

### Example 11: synthesis of intermediate III-3 2-cyano-3-(4-methoxyphenyl)acrylic acid

By reference to the synthesis method of intermediate III-1 2-cyano-3-phenylacrylic acid and substitution of 4-methoxybenzaldehyde for benzaldehyde while other conditions remained unchanged, 0.794 g of intermediate III-3 was obtained with a yield of 86.8%.

ESI-MS: 204.0 [M+H]⁺.

The reaction formula is as follows:

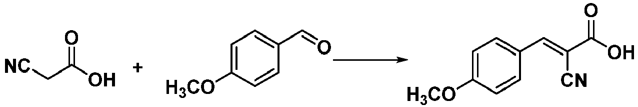

### Example 12: Synthesis of I-9

By reference to the preparation method of compound I-1 and substitution of 2-cyano-3-(4-methoxyphenyl)acrylic acid for 3-phenylacrylic acid as the reaction reagent while other conditions remained unchanged, 0.030 g of the target compound I-9, a yellow solid, was obtained with a yield of 33.7%.

ESI-MS: 648.4 [M+H]⁺, 670.4 [M+Na]⁺.

¹H-NMR (300 MHz, CDCl₃, TMS), *δ* ppm: 0.93 (3H, s), 1.06 (3H, s), 1.12 (3H, s), 1.17 (3H, s), 1.27 (3H, s), 1.34 (3H, s), 1.49 (3H, s), 3.11 (3H, s), 6.01 (1H, s), 6.99 (2H, d, *J* = 8.49 Hz), 7.92 (2H, d, *J* = 8.67 Hz), 8.06 (1H, s), 8.21 (1H, s).

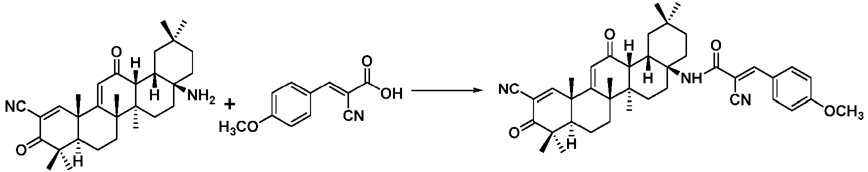

### Example 13: Synthesis of intermediate III-4 2-cyano-3-(4-trifluoromethylphenyl)acrylic acid

By reference to the synthesis method of intermediate III-1 2-cyano-3-phenylacrylic acid and substitution of 4-trifluoromethylbenzaldehyde for benzaldehyde while other conditions remained unchanged, 0.872 g of intermediate III-4 was obtained with a yield of 80.4%.

ESI-MS: 242.0 [M+H]⁺.

The reaction formula is as follows:

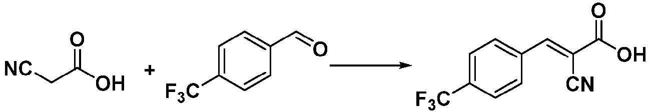

### Example 14: Synthesis of I-10

By reference to the preparation method of compound I-1 and substitution of 2-cyano-3-(4-trifluoromethylphenyl)acrylic acid for 3-phenylacrylic acid as the reaction reagent while other conditions remained unchanged, 0.031 g of the target compound I-10, a white solid, was obtained with a yield of 32.9%.

ESI-MS: 686.4 [M+H]⁺, 684.4 [M-H]⁻.

¹H-NMR (300 MHz, CDCl₃, TMS), *δ* ppm: 0.91 (3H, s), 1.02 (3H, s), 1.08 (3H, s), 1.17 (3H, s), 1.27 (3H, s), 1.44 (3H, s), 1.50 (3H, s), 6.05 (1H, s), 7.68 (2H, d, *J* = 8.07 Hz), 7.88 (2H, *d, J= 8.01* Hz), 8.09 (1H, s).

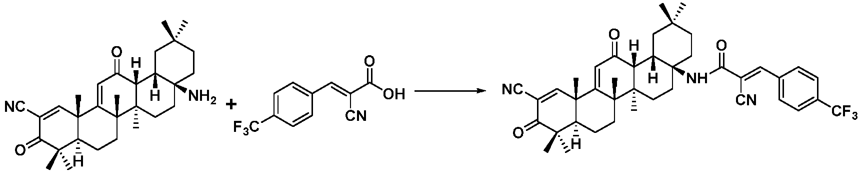

### Example 15: Synthesis of intermediate III-5 2-cyano-3-(4-fluorophenyl)acrylic acid

By reference to the synthesis method of intermediate III-1 2-cyano-3-phenylacrylic acid and substitution of 4-fluorobenzaldehyde for benzaldehyde while other conditions remained unchanged, 0.683 g of intermediate III-5 was obtained with a yield of 79.4%.

ESI-MS: 192.0 [M+H]⁺.

The reaction formula is as follows:

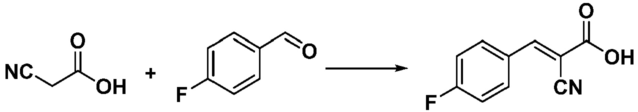

### Example 16: Synthesis of I-11

By reference to the preparation method of compound I-1 and substitution of 2-cyano-3-(4-fluorophenyl)acrylic acid for 3-phenylacrylic acid as the reaction reagent while other conditions remained unchanged, 0.022 g of the target compound I-11, a pale yellow solid, was obtained with a yield of 25.4%.

ESI-MS: 636.4 [M+H]⁺, 658.4 [M+Na]⁺, 635.4 [M-H]⁻.

¹H-NMR (300 MHz, CDCl₃, TMS), *δ* ppm: 0.94 (3H, s), 1.07 (3H, s), 1.13 (3H, s), 1.18 (3H, s), 1.27 (3H, s), 1.44 (3H, s), 1.50 (3H, s), 6.05 (1H, s), 7.21 (4H, m), 8.06 (1H, s), 8.26 (1H, s).

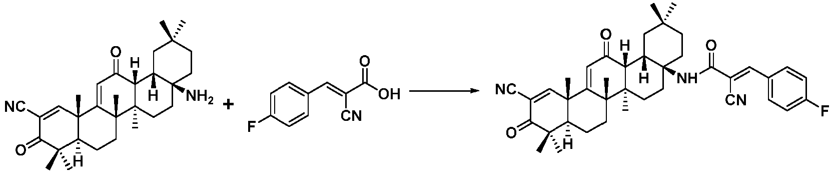

### Example 17: Synthesis of intermediate III-6 2-cyano-3-(4-chlorophenyl)acrylic acid

By reference to the synthesis method of intermediate III-1 2-cyano-3-phenylacrylic acid and substitution of 4-chlorobenzaldehyde for benzaldehyde while other conditions remained unchanged, 0.712 g of intermediate III-6 was obtained with a yield of 76.2%.

ESI-MS: 208.0 [M+H]⁺.

The reaction formula is as follows:

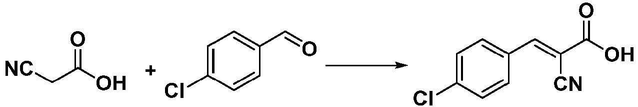

### Example 18: Synthesis of I-12

By reference to the preparation method of compound I-1 and substitution of 2-cyano-3-(4-chlorophenyl)acrylic acid for 3-phenylacrylic acid as the reaction reagent while other conditions remained unchanged, 0.033 g of the target compound I-12, a yellow-white solid, was obtained with a yield of 37.9%.

ESI-MS: 652.3 [M+H]⁺, 674.3 [M+Na]⁺, 650.3 [M-H]⁻.

¹H-NMR (300 MHz, CDCl₃, TMS), *δ* ppm: 0.92 (3H, s), 1.02 (3H, s), 1.08 (3H, s), 1.17 (3H, s), 1.27 (3H, s), 1.44 (3H, s), 1.50 (3H, s), 6.05 (1H, s), 7.42 (2H, d, *J* = 8.47 Hz), 7.82 (2H, d, *J* = 8.51 Hz), 8.12 (1H, s).

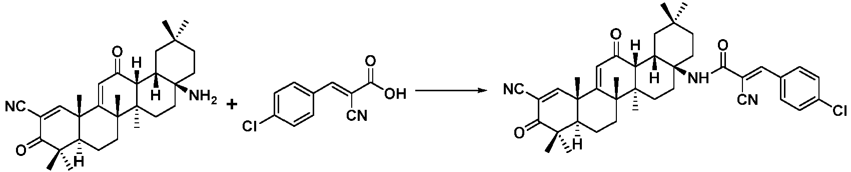

### Example 19: Synthesis of intermediate III-7 3-cyano-3-phenylacrylic acid

0.735 g (5 mmol) of phenylacetonitrile was dissolved in 50 mL of methanol, to which 0.464 g (5 mmol) of glyoxylic acid was added, followed by the addition of 1.035 g (7.5 mmol) of potassium carbonate. The mixture was heated under reflux, the endpoint of the reaction being indicated by TLC results. Following suction filtration, the filter cake was washed with dichloromethane and dissolved in water, the pH being adjusted to 4 with dilute hydrochloric acid, and a white solid was precipitated. Extraction with ethyl acetate (30 mL × 2) was executed, and the organic layers were combined and dried with anhydrous sodium sulfate. Through filtration, rotary evaporation and drying in a constant-temperature drying oven, 0.679 g of intermediate III-7, a white solid, was obtained with a yield of 78.5%.

ESI-MS: 174.0 [M+H]⁺.

The reaction formula is as follows:

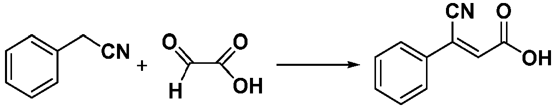

### Example 20: Synthesis of I-13

By reference to the preparation method of compound I-1 and substitution of intermediate III-7 for 3-phenylacrylic acid as the reaction reagent while other conditions remained unchanged, 0.030 g of the target compound I-13, a pale yellow-white solid, was obtained with a yield of 36.2%.

ESI-MS: 618.4 [M+H]⁺, 640.4 [M+Na]⁺.

¹H-NMR (300 MHz, CDCl₃, TMS), *δ* ppm: 0.91 (3H, s), 1.04 (3H, s), 1.06 (3H, s), 1.18 (3H, s), 1.34 (3H, s), 1.42 (3H, s), 1.47 (3H, s), 6.00 (1H, s), 7.03 (1H, s), 7.47 (2H, m), 7.69 (3H, m), 8.04 (1H, s).

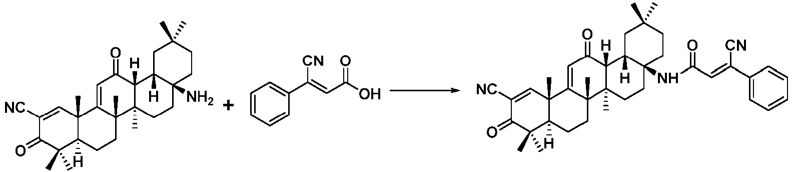

### Example 21: Synthesis of intermediate III-8 3-cyano-3-(4-methylphenyl)acrylic acid

By reference to the synthesis method of intermediate III-7 3-cyano-3-phenylacrylic acid and substitution of 4-methylphenylacetonitrile for phenylacetonitrile as the reaction reagent while other conditions remained unchanged, 0.733 g of intermediate III-8 was obtained with a yield of 78.2%.

ESI-MS: 188.0 [M+H]⁺.

The reaction formula is as follows:

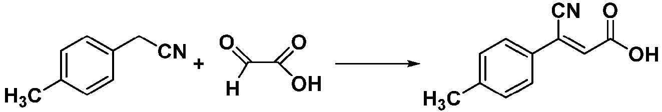

### Example 22: Synthesis of I-14

By reference to the preparation method of compound I-1 and substitution of intermediate III-8 for 3-phenylacrylic acid as the reaction reagent while other conditions remained unchanged, 0.035 g of the target compound I-14, a yellow-white solid, was obtained with a yield of 40.7%.

ESI-MS: 632.4 [M+H]⁺, 654.4 [M+Na]⁺.

¹H-NMR (300 MHz, CDCl₃, TMS), *δ* ppm: 0.90 (3H, s), 1.03 (3H, s), 1.06 (3H, s), 1.18 (3H, s), 1.27 (3H, s), 1.42 (3H, s), 1.47 (3H, s), 2.40 (3H, s), 6.00 (1H, s), 7.01 (1H, s), 7.26 (2H, d, *J* = 8.16 Hz), 7.68 (2H, d, *J* = 7.7 Hz), 8.04 (1H, s).

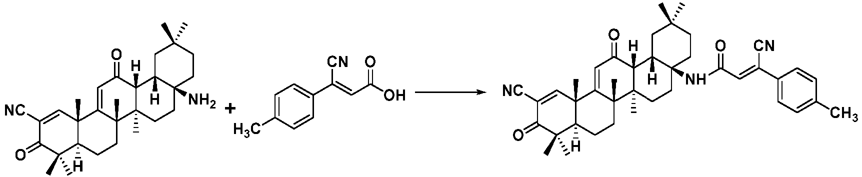

### Example 23: Synthesis of intermediate III-9 3-cyano-3-(4-methoxyphenyl)acrylic acid

By reference to the synthesis method of intermediate III-7 3-cyano-3-phenylacrylic acid and substitution of 4-methoxyphenylacetonitrile for phenylacetonitrile as the reaction reagent while other conditions remained unchanged, 0.757 g of intermediate III-9 was obtained with a yield of 74.5%.

ESI-MS: 204.0 [M+H]⁺.

The reaction formula is as follows:

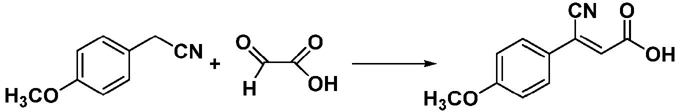

### Example 24: Synthesis of I-15

By reference to the preparation method of compound I-1 and substitution of intermediate III-9 for 3-phenylacrylic acid as the reaction reagent while other conditions remained unchanged, 0.031 g of the target compound I-15, a pale yellow-brown solid, was obtained with a yield of 35.4%.

ESI-MS: 648.4 [M+H]⁺, 670.4 [M+Na]⁺.

¹H-NMR (300 MHz, CDCl₃, TMS), *δ* ppm: 0.89 (3H, s), 0.99 (3H, s), 1.04 (3H, s), 1.17 (3H, s), 1.27 (3H, s), 1.40 (3H, s), 1.45 (3H, s), 3.85 (3H, s), 6.00 (1H, s), 6.94 (2H, d, *J* = 8.76 Hz), 7.03 (1H, s), 7.65 (2H, d, *J* = 8.85 Hz), 8.06 (1H, s).

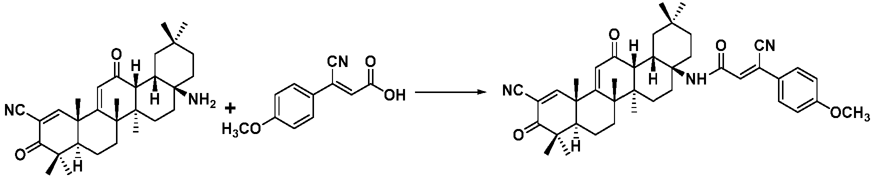

### Example 25: Synthesis of intermediate III-10 3-cyano-3-(4-trifluoromethylphenyl)acrylic acid

By reference to the synthesis method of intermediate III-7 3-cyano-3-phenylacrylic acid and substitution of 4-trifluoromethylphenylacetonitrile for phenylacetonitrile as the reaction reagent while other conditions remained unchanged, 0.833 g of intermediate III-10 was obtained with a yield of 69.1%.

ESI-MS: 242.0 [M+H]⁺.

The reaction formula is as follows:

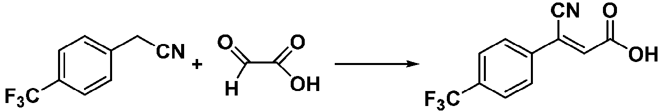

### Example 26: Synthesis of I-16

By reference to the preparation method of compound I-1 and substitution of intermediate III-10 for 3-phenylacrylic acid as the reaction reagent while other conditions remain unchanged, 0.056 g of a pale yellow-brown solid, target compound I-16, was obtained with a yield of 60.2%.

ESI-MS: 686.4 [M+H]⁺, 708.4 [M+Na]⁺.

¹H-NMR (300 MHz, CDCl₃, TMS), *δ* ppm: 0.89 (3H, s), 1.00 (3H, s), 1.06 (3H, s), 1.18 (3H, s), 1.28 (3H, s), 1.42 (3H, s), 1.47 (3H, s), 6.04 (1H, s), 7.15 (1H, s), 7.73 (2H, d, *J =* 8.13 Hz), 7.82 (2H, d, *J* = 7.89 Hz), 8.09 (1H, s).

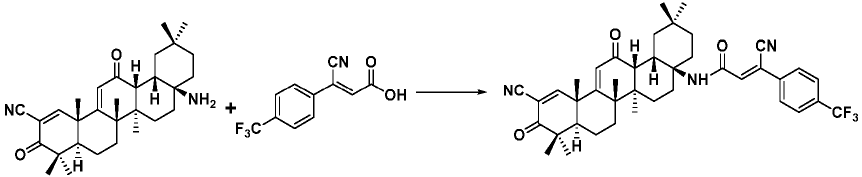

### Example 27: Synthesis of Intermediate III-11 3-cyano-3-(4-fluorophenyl)acrylic acid

By reference to the synthesis method of intermediate III-7 3-cyano-3-phenylacrylic acid and substitution of 4-fluorophenylacetonitrile for phenylacetonitrile as the reaction reagent while other conditions remain unchanged, 0.956 g of intermediate III-11 was obtained with a yield of 81.9%.

ESI-MS: 192.0 [M+H]⁺.

The reaction formula is as follows:

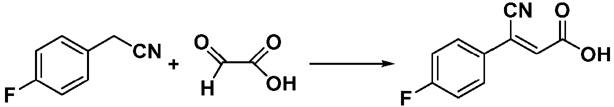

### Example 28: Synthesis of I-17

By reference to the preparation method of compound I-1 and substitution of intermediate III-11 for 3-phenylacrylic acid as the reaction reagent while other conditions remained unchanged, 0.023 g of the target compound I-17, a yellow-white solid, was obtained with a yield of 27.0%.

ESI-MS: 636.4 [M+H]⁺, 658.4 [M+Na]⁺.

¹H-NMR (300 MHz, CDCl₃, TMS), *δ* ppm: 0.90 (3H, s), 1.01 (3H, s), 1.06 (3H, s), 1.18 (3H, s), 1.34 (3H, s), 1.43 (3H, s), 1.49 (3H, s), 6.04 (1H, s), 6.96 (1H, s), 7.15 (2H, d, *J* = 8.49 Hz), 7.70 (2H, d, *J* = 8.76 Hz), 8.09 (1H, s).

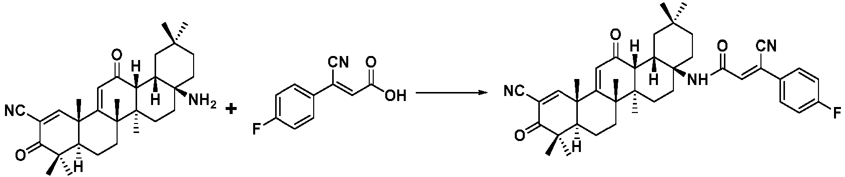

### Example 29: Synthesis of Intermediate III-12 3-cyano-3-(4-chlorophenyl)acrylic acid

By reference to the synthesis method of intermediate III-7 3-cyano-3-phenylacrylic acid and substitution of 4-chlorophenylacetonitrile for phenylacetonitrile as the reaction reagent while other conditions remained unchanged, 0.843 g of the intermediate III-12 was obtained with a yield of 81.2%.

ESI-MS: 208.0 [M+H]⁺.

The reaction formula is as follows:

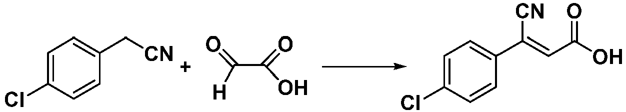

### Example 30: Synthesis of I-18

By reference to the preparation method of compound I-1 and substitution of intermediate III-12 for 3-phenylacrylic acid as the reaction reagent while other conditions remained unchanged, 0.027 g of the target compound I-18, a pale yellow-brown solid, was obtained with a yield of 30.3%.

ESI-MS: 652.3 [M+H]⁺, 674.3 [M+Na]⁺.

¹H-NMR (300 MHz, CDCl₃, TMS), *δ* ppm: 0.88 (3H, s), 0.96 (3H, s), 1.04 (3H, s), 1.17 (3H, s), 1.26 (3H, s), 1.40 (3H, s), 1.45 (3H, s), 6.02 (1H, s), 7.23 (1H, s), 7.40 (2H, d, *J* = 8.40 Hz), 7.64 (2H, d, *J =* 8.43 Hz), 8.09 (1H, s).

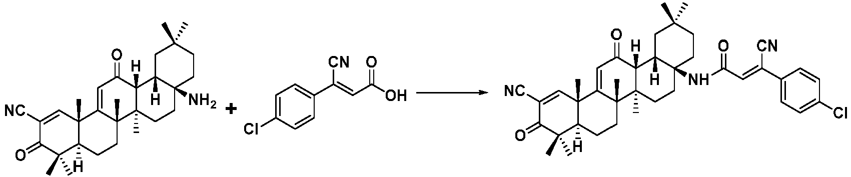

### Example 31: pharmacological experiments on the compounds

The anti-tumor activity of the compounds of the present invention was determined by the method of methyl thiazolyl tetrazolium (MTT), Bardoxolone Methyl (CDDO-Me) being selected as a positive control medicament.

Instruments: ultra-clean bench (SW-CJ-1FD, AIRTECH, Suzhou Antai Airtech Co. Ltd.), constant-temperature CO₂ incubator (3111, Thermo, USA), inverted biomicroscope (IX71, OLYMPUS, Japan), ELISA microplate reader (Model 680, BIO-RAD, USA), platform shaker (Kylin-bell lab Instruments), autoclave (YXO.SG41.280, Shanghai Huaxian), and centrifuge (SIGMA).

Reagents: DMEM (GIBCO), fetal bovine serum (GIBCO), trypsin (SIGMA), and DMSO (SIGMA).

Cell strains: human non-small cell lung cancer cell strain A549, human liver cancer cell strain HepG2, human breast cancer cell strain MCF-7, human renal tubular epithelial cells HK-2, rat embryonic cardiomyocytes H9C2 (all provided by Jiangsu KeyGEN BioTECH Co., Ltd.).

Methods: the cryopreserved cell strains were resuscitated and cultured in a CO₂ incubator at a constant temperature of 37°C with liquid being changed once daily. Plating was carried out when the cell strains were in a good state in the exponential phase. 1 mL of 0.25% trypsin solution was added for digestion for 1-2 min. The cell status was observed under the microscope, and when the adherent cells became round and shrunk, the digestion solution was aspirated and 1-2 mL of a DMEM medium containing 10% fetal bovine serum was added to obtain a cell suspension, on which a cell count was performed. The amount of required cell suspension was calculated on the basis of 5×10⁴ cells per well and the total number of wells. The cell suspension was inoculated on a 96-well plate at 100 µL/well, the surroundings being blocked with PBS, followed by incubation in a CO₂ incubator at a constant temperature of 37°C for 24 h.

The test medicament, positive control medicament CDDO-Me, and blank control group DMSO were prepared in DMEM media to a final concentration of 5 µM/well, each medicament being placed in 3 replicate wells, followed by incubation for 48 h. The MTT reagent was added to the 96-well plate at 10 µL/well to further incubate for 4 h. The medium in the plate was aspirated and 100 µL of DMSO was added to each well, followed by shaking on the platform shaker for 10 min such that the crystals were dissolved. The absorbance of each well was examined by the ELISA microplate reader at a wavelength of 570 nm, and the cell inhibition rate was calculated according to the formula hereinafter. The mean value of three preliminary screening results was taken as the final inhibition rate thereof. Compounds with a preliminary screening inhibition rate greater than 60% were subjected to a concentration gradient screening (5-fold dilution) for the calculation of IC₅₀ values of the test medicaments (calculated by the software graphpad), and the results of three replicate experiments were the final IC₅₀ values of the tested compounds.

Cell inhibition rate (%) = [ (OD value of blank control - OD value of dosing cohort) / OD value of blank control group] × 100%

**Table 1 Inhibitory activity (IC₅₀, µM) of representative compounds of the present invention against proliferation of some tumor cells and cardiomyocytes.**

| Compd | A549 | MCF-7 | HepG2 | HK-2 | H9C2 |
|---|---|---|---|---|---|
| I-1 | 0.599±0.377 | 0.574±0.021 | 0.409±0.016 | 0.799±0.092 | 0.251±0.008 |
| I-2 | 0.549±0.010 | 0.547±0.074 | 0.772±0.063 | 1.388±0.032 | 0.218±0.007 |
| I-3 | 0.367±0.018 | 0.375±0.038 | 0.527±0.133 | 0.700±0.016 | 0.174±0.005 |
| I-4 | 0.402±0.020 | 0.562±0.132 | 0.412±0.006 | 0.516±0.072 | 0.215±0.004 |
| I-5 | 0.788±0.345 | 1.308±0.127 | 0.785±0.096 | 2.825±0.004 | 0.316±0.001 |
| I-6 | 0.588±0.036 | 0.738±0.009 | 0.482±0.002 | 0.727±0.430 | 0.373±0.002 |
| I-7 | 0.315±0.068 | 2.225±0.022 | 2.723±0.036 | 1.783±0.259 | 3.170±1.744 |
| I-8 | 0.329±0.047 | 0.789±0.052 | 0.563±0.023 | 0.423±0.030 | 0.274±0.002 |
| I-9 | 0.624±0.111 | 1.129±0.040 | 0.816±0.014 | 0.748±0.341 | 0.417±0.019 |
| I-10 | 0.362±0.012 | 0.460±0.105 | 0.297±0.011 | 0.234±0.117 | 0.233±0.010 |
| I-11 | 0.609±0.067 | 0.424±0.129 | 0.337±0.012 | 0.308±0.071 | 0.052±0.001 |
| I-12 | 0.761±0.032 | 1.637±0.462 | 0.974±0.058 | 2.432±0.033 | 3.143±1.525 |
| I-13 | 0.414±0.017 | 0.375±0.130 | 0.328±0.013 | 0.473±0.630 | 0.007±0.009 |
| I-14 | 0.337±0.015 | 0.432±0.188 | 0.313±0.035 | 0.939±0.062 | 0.014±0.001 |
| I-15 | 0.658±0.058 | 0.551±0.140 | 0.524±0.032 | 0.350±0.037 | 0.146±0.012 |
| I-16 | 0.888±0.021 | 0.958±0.209 | 0.934±0.011 | 0.618±0.072 | 0.108±0.007 |
| I-17 | 0.345±0.174 | 0.238±0.008 | 0.114±0.004 | 0.229±0.126 | 0.005±~0 |
| I-18 | 0.300±0.013 | 0.500±0.170 | 0.406±0.017 | 0.647±0.021 | 0.002±~0 |
| CDDO-Me | 0.448±0.012 | 0.728±0.288 | 0.326±0.021 | 0.335±0.034 | 0.308±0.010 |

As can be seen from Table 1, most of the compounds of the present invention have greater or comparable inhibitory activities against cardiomyocytes H9C2 than CDDO-Me. The compounds I-1, I-2, I-3, I-4, I-5, I-6, I-7, I-8, I-9, I-12, I-13, I-14, I-16 and I-18 all exhibited lower toxicity on human renal tubular epithelial cells HK-2. In particular, compounds I-7 and I-12 had relatively small inhibitory activities against normal cardiomyocytes H9C2 (IC₅₀ values of 3.176±1.74 µM and 3.143±1.53 µM, respectively), approximately 1/10 of the inhibitory activity of CDDO-Me (IC₅₀ of 0.308±0.01 µM), demonstrating a relatively low myocardial toxicity.

### Example 32 Inhibitory effect of compounds of present invention on tumors in tumor-bearing mice

1) Experimental animals: 72 female ICR mice between 6 and 8 weeks of age; experimental cells: S180 ascitic tumor cells.
   8 animals were used as the normal group, and the rest of the mice were subjected to modeling by the following method.
2) Proliferation and passaging of S180 ascitic tumor cells: S180 ascitic tumor cells cryopreserved at -80°C were thawed in a water bath at 37°C, placed in a 12 mL centrifuge tube, to which an appropriate amount of saline was added, and centrifuged at 1200 rpm for 5 min. The supernatant was discarded, then the cells were resuspended in 300 µL of saline and intraperitoneally injected into an ICR mouse. As the mouse's abdomen swelled up, intraperitoneal injection of the cells in a second ICR mouse was performed, and the cells were ready for inoculation when the abdomen thereof swelled up.
3) Inoculation of S180 tumor cells in the armpit of mice: S180 cells cultured to 2 passages were centrifuged and saline was added to obtain a concentration of 1X10⁷ cells/mL. A volume of 100 µL of the cells were injected subcutaneously into armpit of mice. A mouse tumor-bearing model was developed.

The model mice were divided into a tumor-bearing model group, a compound I-7 5 mg/kg group, a compound I-7 15 mg/kg group, a compound I-12 5 mg/kg group, a compound I-12 15 mg/kg group, a compound I-16 5 mg/kg group, a compound I-16 15 mg/kg group and a CDDO-Me 15 mg/kg group.

There were 8 mice in each group.

4) Dosing: the medicaments were administered intragastrically when the tumor grew out.
Compound I-7 of 5 mg/kg was administered in the compound I-7 5mg/kg group;
compound I-7 of 15 mg/kg was administered in the compound I-7 15mg/kg group;
compound I-12 of 5 mg/kg was administered in the compound I-12 5mg/kg group;
compound I-12 of 15 mg/kg was administered in the compound I-12 15mg/kg group;
compound I-16 of 5 mg/kg was administered in the compound I-16 5mg/kg group;
compound I-16 of 15 mg/kg was administered in the compound I-16 15mg/kg group; and
CDDO-Me of 15 mg/kg was administered in the CDDO-Me 15mg/kg group.

An equal volume of 0.5% croscarmellose sodium was administered to the normal group and the model group.

The dosing was executed for 7 consecutive days. The mice were dissected the next day following the last dosing.

Testing items: the body weight, spleen weight and tumor weight were measured. The spleens were ground on ice, followed by red blood cell lysis and incubation with FITC-CD11b, LY6C, and LY6G antibodies for 15 min, and then tested for CD11b+LY6Chi+ and CD11b+LY6G+ with flow cytometry.

Experimental results are shown in Tables 2 and 3.

**Table 2 Inhibitory effect of the compounds of the present invention on tumors**

| Group | Tumor Mass (g) | Tumor inhibition rate (%) |
|---|---|---|
| Model group | 2.64±0.26 | - |
| Compound I-7 5 mg/kg group | 1.53±0.10^{##}* | 42.17 |
| Compound I-7 15 mg/kg group | 1.45±0.12^{###}** | 45.20 |
| Compound I-12 5 mg/kg group | 1.33±0.11^{###}*** | 49.55 |
| Compound I-12 15 mg/kg group | 0.66±0.14^{###}*** | 74.96 |
| Compound I-16 5 mg/kg group | 1.49 ±0.17^{##}* | 43.69 |
| Compound I-16 15 mg/kg group | 0.96±0.14^{###}*** | 63.60 |
| CDDO-Me 15 mg/kg group | 1.67±0.14^{##} | 36.75 |

| | | |
|---|---|---|
| Note: ^{#}*P* denotes P≤0.05 versus the model group, ^{##}*P* denotes P≤0.01 versus the model group, *^{###}P* denotes P≤0.001 versus the model group; *P denotes P≤0.05 versus the CDDO-Me group, **P denotes P≤0.01 versus the CDDO-Me group, ****P* denotes P≤0.001 versus the CDDO-Me group. | | |

**Table 3 Effect of compounds of present invention on M-MDSC, PMN-MDSC and splenic index**

| Group | M-MDSC (%) | PMN-MDSC (%) | Splenic index (10*mg/g) |
|---|---|---|---|
| Normal group | 0.48±0.08^{###} | 5.44±2.07^{###} | 43.59±7.14^{###} |
| Model group | 1.63±0.33 | 15.53±1.50 | 59.18±5.13 |
| Compound I-7 5 mg/kg group | 0.68±0.22^{###}*** | 8.72±1.13^{###}* | 48.55±4.62^{##}* |
| Compound I-7 15 mg/kg group | 0.59±0.09^{###}*** | 6.34±1.47^{###}*** | 47.03±6.54^{##}* |
| Compound I-12 5 mg/kg group | 0.63±0.13^{###}*** | 8.86±1.53^{###}* | 45.99±7.68^{##}* |
| Compound I-12 15 mg/kg group | 0.38±0.23^{###}*** | 4.82±0.62^{###}*** | 44.37±5.27^{###}** |
| Compound I-16 5 mg/kg group | 0.71±0.08 ^{###}*** | 8.72±2.09^{###}* | 47.86±5.73^{###}* |
| Compound I-16 15 mg/kg group | 0.40±0.07^{###}*** | 6.79±1.55^{###}*** | 46.45±5.20^{###}* |
| CDDO-Me 15 mg/kg group | 1.15±0.12 ^{##} | 10.91±1.62^{###} | 53.61±4.15 |

| | | | |
|---|---|---|---|
| Note: ^{#}*P* denotes P≤0.05 versus the model group, ^{##}*P* denotes P≤0.01 versus the model group, *^{###}P* denotes P≤0.001 versus the model group; **P* denotes P≤0.05 versus the CDDO-Me group, ***P* denotes P≤0.01 versus the CDDO-Me group, ****P* denotes P≤0.001 versus the CDDO-Me group. | | | |

Myeloid-derived suppressor cells (MDSCs) are a population of immature cells of myeloid origin. MDSCs can differentiate into mature macrophages, granulocytes and dendritic cells under normal conditions, but will be aggregated and activated under pathological conditions. In tumors, MDSCs can suppress T-cell immune responses, and can interact with other immune cells and promote, together, the formation of immunosuppression in the tumor microenvironment. In addition, MDSCs can also promote tumor proliferation and metastasis through non-immune pathways such as destruction of extracellular matrix, promoting angiogenesis, etc. Indoleamine 2,3-dioxygenase 1 (IDO1) as one of the key functional markers of MDSCs can catalyze tryptophan metabolism in the tumor microenvironment, leading to the release of soluble kynurenine and downstream metabolites thereof, and inducing immune tolerance by Treg and antigen-presenting cells, thereby resulting in tumor immune escape. As reported in some studies, MDSCs are involved in the progression of pancreatic cancer, breast cancer, brain metastases, etc., and have a great research value as a potential therapeutic target and a reliable prognostic marker.

The example studies of the present invention found that the compounds of the present invention can inhibit the expression of MDSCs and improve the tumor microenvironment, suggesting therapeutic effects thereof on lung cancer, liver cancer, pancreatic cancer, breast cancer, ascitic tumor, brain metastases, etc.

Furthermore, it is to be noted that the compounds selected in Example 32 are representative examples of the target compounds described herein and are not limitations on the present invention.

## Claims

1. A compound of formula I or a pharmaceutically acceptable salt thereof: 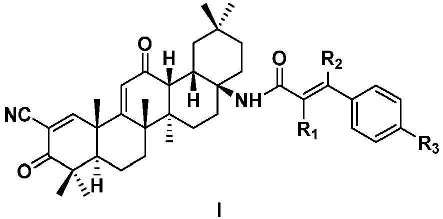 wherein R₁, R₂ and R₃ are respectively and independently selected from the following substituents: H, C₁₋₆ alkyl, C₁₋₆ alkoxy, halogen, cyano, C₁₋₆ alkyl substituted by halogen or cyano.

2. The compound or a pharmaceutically acceptable salt thereof according to claim 1, wherein R₁, R₂ and R₃ are respectively and independently selected from the following substituents: H, C₁₋₃ alkyl, C₁₋₃ alkoxy, halogen, cyano, C₁₋₃ alkyl substituted by halogen or cyano.

3. The compound or a pharmaceutically acceptable salt thereof according to claim 1 or 2, wherein the R₁ and R₂ are respectively and independently selected from H or cyano; R₃ is selected from H, C₁₋₃ alkyl, alkoxy, halogen, C₁₋₃ alkyl substituted by halogen; the alkoxy is an alkoxy containing 1-3 carbon atoms; and the halogen is selected from F, Cl, Br, I.

4. The compound or a pharmaceutically acceptable salt thereof according to claim 1 or 2, wherein the R₁ is H or cyano; R₂ is H or cyano; and R₃ is selected from H, methyl, methoxy, trifluoromethyl, Cl or F.

5. The compound or a pharmaceutically acceptable salt thereof according to claim 1 or 2, wherein the R₁ is H or cyano; R₂ is H or cyano; and R₃ is Cl, H or trifluoromethyl.

6. The compound or a pharmaceutically acceptable salt thereof according to claim 1 or 2, wherein the compound is selected from:
**3**-phenyl-***N*-(2**-cyano-**3,12**-dioxoolean-**1,9(11)**-dien-**19**-yl) acrylamide,
**3-(4**-methylphenyl)-*N***-(2**-cyano-**3,12**-dioxoolean-**1,9(11)**-dien-**19**-yl) acrylamide,
**3-(4**-methoxyphenyl)-*N*-(2-cyano-**3,12**-dioxoolean-**1,9(11)**-dien-**19**-yl) acrylamide,
**3-(4**-trifluoromethylphenyl)-***N*-(2**-cyano-**3,12**-dioxoolean-**1,9(11)**-dien-**19**-yl) acrylamide,
**3-(4**-fluorophenyl)-***N*-(2**-cyano-**3,12**-dioxoolean-**1,9(11)**-dien-**19**-yl) acrylamide,
**3-(4**-chlorophenyl)-***N*-(2**-cyano-**3,12**-dioxoolean-**1,9(11)**-dien-**19**-yl) acrylamide,
**2**-cyano-**3**-ylphenyl-***N*-(2**-cyano-**3,12**-dioxoolean-**1,9(11)**-dien**-19**-yl) acrylamide,
**2**-cyano-**3-(4**-methylphenyl)-***N*-(2**-cyano-**3,12**-dioxoolean-**1,9(11)**-dien-**19**-yl) acrylamide,
**2**-cyano-**3-(4**-methoxyphenyl)-***N*-(2**-cyano-**3,12**-dioxoolean-**1,9(11)**-dien-**19**-yl) acrylamide,
**2**-cyano-**3-(4**-trifluoromethylphenyl)-***N*-(2**-cyano-**3,12**-dioxoolean-**1,9(11)**-dien-**19**-yl) acrylamide,
**2**-cyano-**3-(4**-fluorophenyl)-***N*-(2**-cyano-**3,12**-dioxoolean-**1,9(11)**-dien-**19**-yl) acrylamide,
**2**-cyano-**3-(4**-chlorophenyl)-***N*-(2**-cyano-**3,12**-dioxoolean-**1,9(11)**-dien-**19**-yl) acrylamide,
**3**-phenyl-**3**-cyano-***N***-(**2**-cyano-**3,12**-dioxoolean-**1,9(11)**-dien-**19**-yl) acrylamide,
**3-(4**-methylphenyl)-**3**-cyano-***N*-(2**-cyano-**3,12**-dioxoolean-**1,9(11)**-dien-**19**-yl) acrylamide,
**3-(4**-methoxyphenyl)-**3**-cyano-***N*-(2**-cyano-**3,12**-dioxoolean-**1,9(11)**-dien-**19**-yl) acrylamide,
**3-(4**-trifluoromethylphenyl)-**3**-cyano-***N*-(2**-cyano-**3,12**-dioxoolean-**1,9(11)**-dien-**19**-yl) acrylamide,
**3-(4**-fluorophenyl)-**3**-cyano-***N*-(2**-cyano-**3,12**-dioxoolean-**1,9(11)**-dien-**19**-yl) acrylamide, and
**3-(4**-chlorophenyl)-**3**-cyano-***N*-(2**-cyano-**3,12**-dioxoolean-**1,9(11)**-dien-**19**-yl) acrylamide.

7. A method for synthesizing the compound of formula I according to any one of claims 1 to 6, comprising: reacting formula II with formula III via a condensation reaction to obtain the compound of formula I, 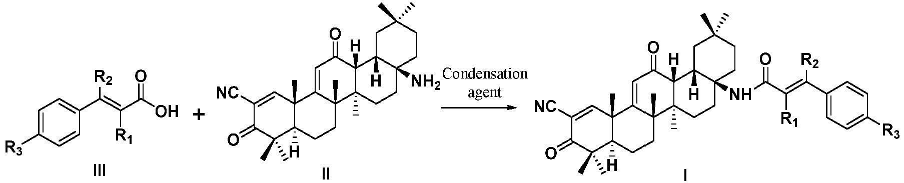 where in the R₁, R₂ and R₃ are as defined in any one of claims 1-6, and the condensation agent is one or more selected from dicyclohexylcarbodiimide, N,N-diisopropylcarbodiimide, 1-ethyl-(3-dimethylaminopropyl) carbodiimide, benzotriazol-1-yloxytris(dimethylamino)phosphonium hexafluorophosphate or benzotriazol-1-yloxytripyrrolidinophosphonium hexafluorophosphate.

8. The synthesis method according to claim 7, wherein a method for synthesizing the formula II comprises: 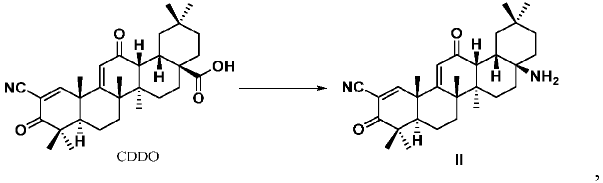 subjecting CDDO to Curtius rearrangement, so as to obtain a derivative aminated at the C-17 position as the formula II.

9. A pharmaceutical composition, comprising the compound or a pharmaceutically acceptable salt thereof according to any one of claims 1-6, and one or more pharmaceutically acceptable pharmaceutical adjuvants.

10. The compound or a pharmaceutically acceptable salt thereof according to any one of claims 1-6 or the pharmaceutical composition according to claim 9, for use in treating tumor.

11. The compound or a pharmaceutically acceptable salt thereof or the pharmaceutical composition for use according to claim 10, wherein the tumor is selected from lung cancer, liver cancer, ascitic tumor, brain metastases, colon cancer, pancreatic cancer, breast cancer, prostatic cancer, brain cancer, ovarian carcinoma, cervical cancer, testicular cancer, kidney cancer, head and neck cancer, lymphoma, melanoma or leukemia.

12. The compound or a pharmaceutically acceptable salt thereof or the pharmaceutical composition for use according to claim 10, wherein the tumor is selected from lung cancer, liver cancer, breast cancer, ascitic tumor, pancreatic cancer, and brain metastases.

13. The compound or a pharmaceutically acceptable salt thereof or the pharmaceutical composition for use according to claim 10, wherein the tumor is selected from non-small cell lung cancer, liver cancer, breast cancer, and ascitic tumor.

## Patentansprüche

1. Verbindung der Formel I oder ein pharmazeutisch unbedenkliches Salz davon: 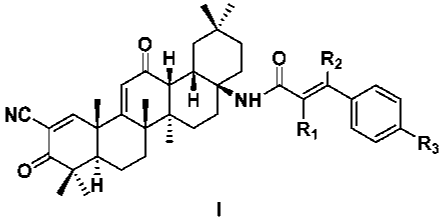 wobei R₁, R₂ und R₃ jeweils und unabhängig voneinander aus den folgenden Substituenten ausgewählt sind: H, C₁₋₆-Alkyl, C₁₋₆-Alkoxy, Halogen, Cyano, durch Halogen oder Cyano substituiertes C₁₋₆-Alkyl.

2. Verbindung oder ein pharmazeutisch unbedenkliches Salz davon nach Anspruch 1, wobei R₁, R₂ und R₃ jeweils und unabhängig voneinander aus den folgenden Substituenten ausgewählt sind: H, C₁₋₃-Alkyl, C₁₋₃-Alkoxy, Halogen, Cyano, durch Halogen oder Cyano substituiertes C₁₋₃-Alkyl.

3. Verbindung oder ein pharmazeutisch unbedenkliches Salz davon nach Anspruch 1 oder 2, wobei R₁ und R₂ jeweils und unabhängig voneinander aus H oder Cyano ausgewählt sind; R₃ aus H, C₁₋₃-Alkyl, Alkoxy, Halogen, mit Halogen substituiertem C₁₋₃-Alkyl ausgewählt ist; wobei das Alkoxy ein 1 bis 3 Kohlenstoffatome enthaltendes Alkoxy ist und das Halogen aus F, Cl, Br, I ausgewählt ist.

4. Verbindung oder ein pharmazeutisch unbedenkliches Salz davon nach Anspruch 1 oder 2, wobei R₁ H oder Cyano ist; R₂ H oder Cyano ist; und R₃ aus H, Methyl, Methoxy, Trifluormethyl, Cl oder F ausgewählt ist.

5. Verbindung oder ein pharmazeutisch unbedenkliches Salz davon nach Anspruch 1 oder 2, wobei R₁ H oder Cyano ist; R₂ H oder Cyano ist; und R₃ Cl, H oder Trifluormethyl ist.

6. Verbindung oder ein pharmazeutisch unbedenkliches Salz davon nach Anspruch 1 oder 2, wobei die Verbindung ausgewählt ist aus:
3-Phenyl-N-(2-cyano-3,12-dioxoolean-1,9(11)-dien-19-yl)acrylamid,
3-(4-Methylphenyl)-N-(2-cyano-3,12-dioxoolean-1,9(11)-dien-19-yl)acrylamid,
3-(4-Methoxyphenyl)-N-(2-cyano-3,12-dioxoolean-1,9(11)-dien-19-yl)acrylamid,
3-(4-Trifluormethylphenyl)-N-(2-cyano-3,12-dioxoolean-1,9(11)-dien-19-yl)acrylamid,
3-(4-Fluorphenyl)-N-(2-cyano-3,12-dioxoolean-1,9(11)-dien-19-yl)acrylamid,
3-(4-Chlorphenyl)-N-(2-cyano-3,12-dioxoolean-1,9(11)-dien-19-yl)acrylamid,
2-Cyano-3-ylphenyl-N-(2-cyano-3,12-dioxoolean-1,9(11)-dien-19-yl)acrylamid,
2-Cyano-3-(4-methylphenyl)-N-(2-cyano-3,12-dioxoolean-1,9(11)-dien-19-yl)acrylamid,
2-Cyano-3-(4-methoxyphenyl)-N-(2-cyano-3,12-dioxoolean-1,9(11)-dien-19-yl)acrylamid,
2-Cyano-3-(4-trifluormethylphenyl)-N-(2-cyano-3,12-dioxoolean-1,9(11)-dien-19-yl)acrylamid,
2-Cyano-3-(4-fluorphenyl)-N-(2-cyano-3,12-dioxoolean-1,9(11)-dien-19-yl)acrylamid,
2-Cyano-3-(4-chlorphenyl)-N-(2-cyano-3,12-dioxoolean-1,9(11)-dien-19-yl)acrylamid,
3-Phenyl-3-cyano-N-(2-cyano-3,12-dioxoolean-1,9(11)-dien-19-yl)acrylamid,
3-(4-Methylphenyl)-3-cyano-N-(2-cyano-3,12-dioxoolean-1,9(11)-dien-19-yl)acrylamid,
3-(4-Methoxyphenyl)-3-cyano-N-(2-cyano-3,12-dioxoolean-1,9(11)-dien-19-yl)acrylamid,
3-(4-Trifluormethylphenyl)-3-cyano-N-(2-cyano-3,12-dioxoolean-1,9(11)-dien-19-yl)acrylamid,
3-(4-Fluorphenyl)-3-cyano-N-(2-cyano-3,12-dioxoolean-1,9(11)-dien-19-yl)acrylamid, und
3-(4-Chlorphenyl)-3-cyano-N-(2-cyano-3,12-dioxoolean-1,9(11)-dien-19-yl)acrylamid.

7. Verfahren zum Synthetisieren der Verbindung der Formel I nach einem der Ansprüche 1 bis 6, umfassend: Umsetzen der Formel II mit Formel III über eine Kondensationsreaktion, um die Verbindung der Formel I zu erhalten, 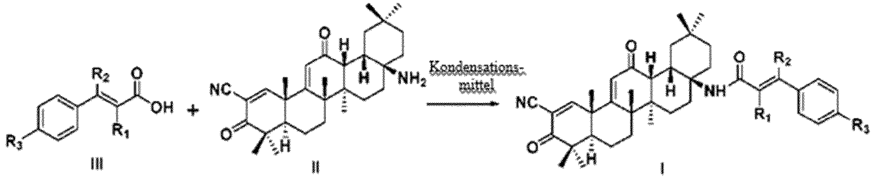 wobei
in der R₁, R₂ und R₃ wie in einem der Ansprüche 1 bis 6 definiert sind und das Kondensationsmittel eines oder mehrere ist, ausgewählt aus Dicyclohexylcarbodiimid, N,N-Diisopropylcarbodiimid, 1-Ethyl-(3-dimethylaminopropyl)carbodiimid, Benzotriazol-1-yloxytris(dimethylamino)phosphoniumhexafluorophosphat oder Benzotriazol-1-yloxytripyrrolidinophosphoniumhexafluorophosphat.

8. Syntheseverfahren nach Anspruch 7, wobei ein Verfahren zum Synthetisieren der Formel II umfasst: 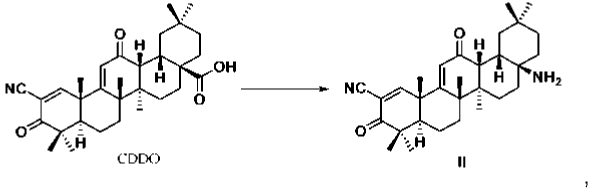 Unterziehen von CDDO einer Curtius-Umlagerung, um so ein an der C-17-Position aminiertes Derivat der Formel II zu erhalten.

9. Pharmazeutische Zusammensetzung, umfassend die Verbindung oder ein pharmazeutisch unbedenkliches Salz davon nach einem der Ansprüche 1 bis 6 und ein oder mehrere pharmazeutisch unbedenkliche pharmazeutische Hilfsstoffe.

10. Verbindung oder ein pharmazeutisch unbedenkliches Salz davon nach einem der Ansprüche 1 bis 6 oder die pharmazeutische Zusammensetzung nach Anspruch 9 zur Verwendung bei der Behandlung von Tumoren.

11. Verbindung oder ein pharmazeutisch unbedenkliches Salz davon oder die pharmazeutische Zusammensetzung zur Verwendung nach Anspruch 10, wobei der Tumor ausgewählt ist aus Lungenkrebs, Leberkrebs, Aszites-Tumor, Hirnmetastasen, Darmkrebs, Bauchspeicheldrüsenkrebs, Brustkrebs, Prostatakrebs, Hirnkrebs, Eierstockkarzinom, Gebärmutterhalskrebs, Hodenkrebs, Nierenkrebs, Kopf- und Halskrebs, Lymphom, Melanom oder Leukämie.

12. Verbindung oder ein pharmazeutisch unbedenkliches Salz davon oder die pharmazeutische Zusammensetzung zur Verwendung nach Anspruch 10, wobei der Tumor ausgewählt ist aus Lungenkrebs, Leberkrebs, Brustkrebs, Aszites-Tumor, Bauchspeicheldrüsenkrebs und Hirnmetastasen.

13. Verbindung oder ein pharmazeutisch unbedenkliches Salz davon oder die pharmazeutische Zusammensetzung zur Verwendung nach Anspruch 10, wobei der Tumor ausgewählt ist aus nicht-kleinzelligem Lungenkrebs, Leberkrebs, Brustkrebs und Aszites-Tumor.

## Revendications

1. Composé de formule I ou sel pharmaceutiquement acceptable de celui-ci : 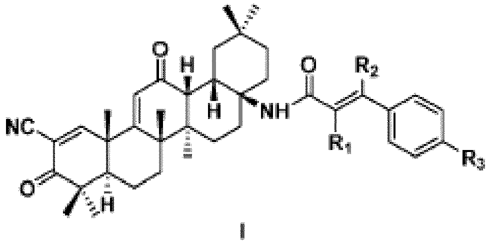 dans lequel R₁, R₂ et R₃ sont respectivement et indépendamment sélectionnés parmi les substituants suivants : H, un alkyle en C₁₋₆, un alcoxy en C₁₋₆, un halogène, un cyano, un alkyle en C₁₋₆ substitué avec un halogène ou un cyano.

2. Composé ou sel pharmaceutiquement acceptable de celui-ci selon la revendication 1, dans lequel R₁, R₂ et R₃ sont respectivement et indépendamment sélectionnés parmi les substituants suivants : H, un alkyle en C₁₋₃, un alcoxy en C₁₋₃, un halogène, un cyano, un alkyle en C₁₋₃ substitué avec un halogène ou un cyano.

3. Composé ou sel pharmaceutiquement acceptable de celui-ci selon la revendication 1 ou 2, dans lequel R₁ et R₂ sont respectivement et indépendamment sélectionnés parmi H ou un cyano ; R₃ est sélectionné parmi H, un alkyle en C₁₋₃, un alcoxy, un halogène, un alkyle en C₁₋₃ substitué avec un halogène ; l'alcoxy est un alcoxy contenant 1 à 3 atomes de carbone ; et l'halogène est sélectionné parmi F, Cl, Br, I.

4. Composé ou sel pharmaceutiquement acceptable de celui-ci selon la revendication 1 ou 2, dans lequel R₁ est H ou un cyano ; R₂ est H ou un cyano ; et R₃ est sélectionné parmi H, un méthyle, un méthoxy, un trifluorométhyle, Cl ou F.

5. Composé ou sel pharmaceutiquement acceptable de celui-ci selon la revendication 1 ou 2, dans lequel R₁ est H ou un cyano ; R₂ est H ou un cyano ; et R₃ est Cl, H ou un trifluorométhyle.

6. Composé ou sel pharmaceutiquement acceptable de celui-ci selon la revendication 1 ou 2, dans lequel le composé est sélectionné parmi :
le 3-phényl-*N*-(2-cyano-3,12-dioxooléan-1,9(11)-diène-19-yl)acrylamide,
le 3-(4-méthylphényl)-*N*-(2-cyano-3,12-dioxooléan-1,9(11)-diène-19-yl)acrylamide,
le 3-(4-méthoxyphényl)-*N*-(2-cyano-3,12-dioxooléan-1,9(11)-diène-19-yl)acrylamide,
le 3-(4-trifluorométhylphényl)-*N*-(2-cyano-3,12-dioxooléan-1,9(11)-diène-19-yl)acrylamide,
le 3-(4-fluorophényl)-*N*-(2-cyano-3,12-dioxooléan-1,9(11)-diène-19-yl)acrylamide,
le 3-(4-chlorophényl)-*N*-(2-cyano-3,12-dioxooléan-1,9(11)-diène-19-yl)acrylamide,
le 2-cyano-3-ylphényl-*N*-(2-cyano-3,12-dioxooléan-1,9(11)-diène-19-yl)acrylamide,
le 2-cyano-3-(4-méthylphényl)-*N*-(2-cyano-3,12-dioxooléan-1,9(11)-diène-19-yl)acrylamide,
le 2-cyano-3-(4-méthoxyphényl)-*N*-(2-cyano-3,12-dioxooléan-1,9(11)-diène-19-yl)acrylamide,
le 2-cyano-3-(4-trifluorométhylphényl)-*N*-(2-cyano-3,12-dioxooléan-1,9(11)-diène-19-yl)acrylamide,
le 2-cyano-3-(4-fluorophényl)-*N*-(2-cyano-3,12-dioxooléan-1,9(11)-diène-19-yl)acrylamide,
le 2-cyano-3-(4-chlorophényl)-*N*-(2-cyano-3,12-dioxooléan-1,9(11)-diène-19-yl)acrylamide,
le 3-phényl-3-cyano-*N*-(2-cyano-3,12-dioxooléan-1,9(11)-diène-19-yl)acrylamide,
le 3-(4-méthylphényl)-3-cyano-*N*-(2-cyano-3,12-dioxooléan-1,9(11)-diène-19-yl)acrylamide,
le 3-(4-méthoxyphényl)-3-cyano-*N*-(2-cyano-3,12-dioxooléan-1,9(11)-diène-19-yl)acrylamide,
le 3-(4-trifluorométhylphényl)-3-cyano-*N*-(2-cyano-3,12-dioxooléan-1,9(11)-diène-19-yl)acrylamide,
le 3-(4-fluorophényl)-3-cyano-*N*-(2-cyano-3,12-dioxooléan-1,9(11)-diène-19-yl)acrylamide, et
le 3-(4-chlorophényl)-3-cyano-*N*-(2-cyano-3,12-dioxooléan-1,9(11)-diène-19-yl)acrylamide.

7. Procédé de synthèse du composé de formule I selon l'une quelconque des revendications 1 à 6, comprenant : la réaction de la formule II avec la formule III par une réaction de condensation pour obtenir le composé de formule I, 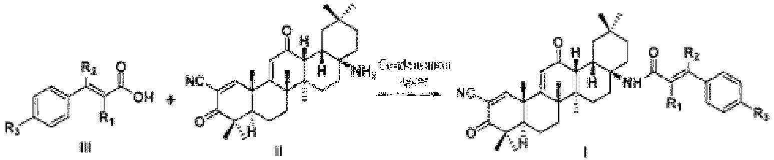 où R₁, R₂ et R₃ sont tels que définis dans l'une quelconque des revendications 1 à 6, et l'agent de condensation est un ou plusieurs sélectionnés parmi le dicyclohexylcarbodiimide, le *N*,*N*-diisopropylcarbodiimide, le 1-éthyl-(3-diméthylaminopropyl)carbodiimide, l'hexafluorophosphate de benzotriazol-1-yloxytris(diméthylamino)phosphonium ou l'hexafluorophosphate de benzotriazol-1-yloxytripyrrolidinophosphonium.

8. Procédé de synthèse selon la revendication 7, dans lequel un procédé de synthèse de la formule II comprend : 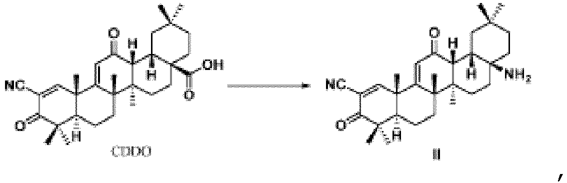 la soumission du CDDO à un réarrangement de Curtius, de manière à obtenir un dérivé aminé en position C-17 de formule II.

9. Composition pharmaceutique, comprenant le composé ou un sel pharmaceutiquement acceptable de celui-ci selon l'une quelconque des revendications 1 à 6, et un ou plusieurs adjuvants pharmaceutiques pharmaceutiquement acceptables.

10. Composé ou sel pharmaceutiquement acceptable de celui-ci selon l'une quelconque des revendications 1 à 6 ou composition pharmaceutique selon la revendication 9, pour une utilisation dans le traitement d'une tumeur.

11. Composé ou sel pharmaceutiquement acceptable de celui-ci ou composition pharmaceutique pour une utilisation selon la revendication 10, dans lequel la tumeur est sélectionnée parmi un cancer du poumon, un cancer du foie, une tumeur ascitique, les métastases cérébrales, un cancer du côlon, un cancer du pancréas, un cancer du sein, un cancer de la prostate, un cancer du cerveau, un cancer de l'ovaire, un cancer du col de l'utérus, un cancer des testicules, un cancer du rein, un cancer de la tête et du cou, un lymphome, un mélanome ou une leucémie.

12. Composé ou sel pharmaceutiquement acceptable de celui-ci ou composition pharmaceutique pour une utilisation selon la revendication 10, dans lequel la tumeur est sélectionnée parmi un cancer du poumon, un cancer du foie, un cancer du sein, une tumeur ascitique, un cancer du pancréas et des métastases cérébrales.

13. Composé ou sel pharmaceutiquement acceptable de celui-ci ou composition pharmaceutique pour une utilisation selon la revendication 10, dans lequel la tumeur est sélectionnée parmi un cancer du poumon non à petites cellules, un cancer du foie, un cancer du sein et une tumeur ascitique.
